(19) Europäisches Patentamt
European Patent Office
Office européen des brevets

(11) **EP 4 285 849 A1**

(12) **EUROPEAN PATENT APPLICATION**

(43) Date of publication:
**06.12.2023  Bulletin 2023/49**

(21) Application number: **23172909.6**

(22) Date of filing: **11.05.2023**

(51) International Patent Classification (IPC):
**A61B 18/02** (2006.01)    A61B 18/00 (2006.01)

(52) Cooperative Patent Classification (CPC):
**A61B 18/02;** A61B 18/00; A61B 2018/00005;
A61B 2018/00011; A61B 2018/00577;
A61B 2018/00654; A61B 2018/00744;
A61B 2018/00791; A61B 2018/00797;
A61B 2018/0212; A61B 2018/0262;
A61B 2018/0293

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.05.2022   US 202217828128**

(71) Applicant: **IceCure Medical Ltd.**
**3088900 Caesarea (IL)**

(72) Inventors:
• **KAUFMAN, Shai**
  **4584500 Nir Eliyahu (IL)**

• **MUCHNIK, Naum**
  **2067633 83 Hermon Street, Yokneam Illit (IL)**
• **BRUDNER, Dori**
  **61 Ha'Shomer Street, Zichron Yaakov (IL)**
• **LASKER, Lior**
  **2 Elyahu Hakim Street, Apt. 20, Haifa (IL)**
• **HILLELI, Ron**
  **3095306 3 Af Al Pi Chen Street, Zichron Yaacov (IL)**

(74) Representative: **Beck Greener LLP**
**Fulwood House
12 Fulwood Place
London WC1V 6HR (GB)**

(54)    **CRYOGENIC SYSTEM WITH MULTIPLE SUBMERGED PUMPS**

(57)    Apparatus, consisting of a single insulated container, configured to contain a cryogen. The apparatus also has a first pump and a second pump, both disposed in the single insulated container and configured to pump the cryogen from the single insulated container while immersed in the cryogen. A first probe and a second probe, having respective first and second proximal ends, are coupled to receive concurrently the cryogen pumped by the first pump and the second pump, respectively. The probes have respective first and second distal ends, which are chilled by the cryogen received through the first and second proximal ends and which are configured to be inserted into a body of a living subject.

FIG. 2B

EP 4 285 849 A1

## Description

### FIELD OF THE INVENTION

This invention relates generally to cryogenic systems, and specifically to systems having more than one pump.

### BACKGROUND OF THE INVENTION

There are many cryogenic systems known in the art. Examples of some systems are described below.

U.S. Patent 7,083,612 to Littrup et al. describes a cryotherapy system having multiple cryoprobes, each of which has a shaft with a closed distal end adapted for insertion into a body.

U.S. Patent 7,192,426 to Baust et al. describes a cryosurgical system for supplying cryogen to a probe. The system includes a container filled with cryogen that has bellows of a pump submerged within the cryogen.

U.S. Patent 7,422,583 and U.S. Patent Application 2004/0210212 to Maurice describe cryosurgical apparatus including a cryoprobe having a cooling portion and an electrically conductive first portion in the region of the cooling portion. The cryoprobe also has a removable sheath.

U.S. Patent 8,784,409 to Robilotto et al. describes a device that is a closed or semi-closed system in which liquid cryogen is contained in both supply and return stages.

U.S. Patent 9,316,215 to Mackey describes a multiple pump system that includes a fluid tank and a multiple pump vessel connected to the fluid tank.

U.S. Patent 11,026,737 to Baust et al. describes cooling an object, including living tissue, to freezing or cryogenic temperatures by placing the object in thermal communication with sub-cooled supercritical nitrogen.

U.S. Patent 11,060,778 to Jankowsky et al. describes a universal controller for integration of cryogenic equipment, requiring different control mechanisms, onto a single operating platform. The universal controller may include a power supply element that is configured to simultaneously drive a plurality of cryogenic devices that have different power supply requirements.

U.S. Patent 11,266,458 to Perron describes a surgical cryoablation system comprising a valve having a valve inlet and a valve outlet. The valve inlet is connectable to a source of cryogenic fluid at a pressure of greater than 4000 psi and the valve outlet is connectable to a cryoablation probe.

U.S. Patent Application 2007/0244474 to DeLonzor et al. describes a cryosurgical system using a low-pressure liquid nitrogen supply. The system is stated to require only 0.5 to 15 bar of pressure to provide adequate cooling power for treatment of typical breast lesions. The pressure may be provided by supplying lightly pressurized air into a Dewar, by heating a small portion of the nitrogen in the Dewar, or with a small low pressure pump.

U.S. Patent Application 2008/0125764 to Vancelette et al. describes a cryosurgical system providing for temperature control of individual cryoprobes so as to simplify and increase treatment flexibility in cryoablation procedures. The cryosurgical system provides individual control of multiple cryoprobes in a closed-loop refrigeration circuit. The individual control allows the simultaneous use of multiple cryoprobes in a procedure.

U.S. Patent Application 2015/0126987 to Semenov et al. describes a method for feeding a cryogenic agent to a cryogenic instrument, by connecting the cryogenic instrument to a reservoir with the cryogenic agent via a cryogenic conduit, and feeding the cryogenic agent from the reservoir to the cryogenic instrument via the cryogenic conduit.

U.S. Patent Application 2016/0249970 to Yu et al. describes an endovascular near critical fluid based cryoablation catheter for creating an elongated lengthwise-continuous lesion in tissue. The catheter comprises an elongated shaft, a flexible distal tissue treatment section, and a distal tip.

U.S. Patent Application 2020/0121498 to Baust et al. describes a cryogenic medical device for delivery of subcooled liquid cryogen to various configurations of cryoprobes that are designed for the treatment of damaged, diseased, cancerous or other unwanted tissues. The device is a closed or semi-closed system in which the liquid cryogen is contained in both the supply and return stages.

U.S. Patent Application 2021/0177482 to Tegg et al. describes a medical device that can comprise a balloon, and a movable manifold. The movable manifold comprises a first plurality of openings and is inside the balloon and is configured to distribute a fluid within the balloon.

U.S. Patent Application 2021/0244457 to Hilleli et al. describes a probe containing a lumen and having a distal end configured to contact tissue of a living subject. A temperature sensor is located at the distal end, and a pump, having a pump motor, is coupled to deliver a cryogenic fluid through the lumen to the distal end of the probe and to receive the cryogenic fluid returning from the probe.

### SUMMARY OF THE INVENTION

An embodiment of the present invention provides apparatus, consisting of:

a single insulated container, configured to contain a cryogen;

a first pump and a second pump, both disposed in the single insulated container and configured to pump the cryogen from the single insulated container while immersed in the cryogen; and

a first probe and a second probe, having respective first and second proximal ends coupled to receive concurrently the cryogen pumped by the first pump and the second pump, respectively, and having respective first and second distal ends, which are chilled by the cryogen received through the first and second proximal ends and are configured to be inserted into a body of a living subject.

Typically, the first pump and the second pump are respectively selected from a group consisting of a bellows pump, a plunger pump, and a piston pump.

In a disclosed embodiment the first pump and the second pump are not connected.

In a further disclosed embodiment the cryogen pumped by the first pump is at a first rate of flow, and the cryogen pumped by the second pump is at a second rate of flow, and the first and second rates of flow are independent of each other.

Typically, the first distal end has a first temperature sensor and the second distal end has a second temperature sensor, and the first rate of flow is in response to a first temperature measured by the first temperature sensor and the second rate of flow is in response to a second temperature measured by the second temperature sensor.

In a yet further disclosed embodiment the apparatus includes a processor configured to operate the first pump and the second pump.

There is also provided, according to an embodiment of the present invention, a method, consisting of:

providing a single insulated container configured to contain a cryogen;

disposing a first pump and a second pump in the single insulated container, and configuring the pumps to pump the cryogen from single insulated container while immersed in the cryogen; and

coupling a first probe and a second probe respectively to the first pump and the second pump, wherein the first probe and the second probe have respective first and second proximal ends coupled to receive concurrently the cryogen pumped by the first pump and the second pump, respectively, and have respective first and second distal ends, which are chilled by the cryogen received through the first and second proximal ends and are configured to be inserted into a body of a living subject

The present disclosure will be more fully understood from the following detailed description of the embodiments thereof, taken together with the drawings, in which:

## BRIEF DESCRIPTION OF THE DRAWINGS

Fig. 1 is a schematic illustration of an apparatus being used for a multi-probe cryogenic procedure, according to an embodiment of the present invention;

Fig. 2A is a schematic diagram illustrating the structure and operation of a console, and Fig. 2B is a schematic diagram of two piston pumps operating in the console, according to an embodiment of the present invention;

Fig. 3 is a schematic block diagram indicating how a processor controls the flow rate of a pump, according to an embodiment of the present invention; and

Fig. 4 is a flowchart of operations taken by a processor in executing an algorithm to operate a pump during the procedure of Fig. 1, according to an embodiment of the present invention.

## DETAILED DESCRIPTION OF EMBODIMENTS

Overview

A typical cryogenic medical procedure for treatment of a patient's tumor involves insertion of a distal end of a probe into the tumor. The proximal end of the probe is connected to a cryogen pumping system external to the patient, and the system then pumps a cryogen into the probe's distal end, so as to freeze, and thus kill, the tumor. The freezing from the single probe creates an approximately spherical iceball around the probe distal end that encompasses the tumor.

For tumors that are not encompassed by a spherical iceball, or where the iceball encases non-tumorous tissue, e.g., elongated tumors, it may be necessary to use and independently operate one or more further probes. Such further probes enable the iceball to be shaped according to the shape of the tumor.

However, each further probe requires a respective external cryogen pumping system, and the provision of multiple external cryogen pumping systems during a medical procedure is cumbersome and costly.

Embodiments of the present invention address the problem of multiple pumping systems by providing a single system that comprises multiple pumps, each of the pumps being able to be connected to the proximal end of a respective probe. The distal ends of the probes can be inserted independently into the tumor, and once inserted, the distal ends can be cooled independently. In contrast to the prior art cryogen pumping systems referred to above, wherein each pumping system is retained in its own insulated container, the single system of the present invention uses just one insulated container. The one insulated container, typically a Dewar, is configured to retain two or more cryogenic pumping systems. However, as for the prior art systems, each of the pumping systems of the present invention may be operated independently of each other.

Having just one insulated container for the multiple pumping systems reduces the time that needs to be spent on filling and emptying cryogen from the systems. In addition, for multiple pumping systems in separate insulated containers, there is the problem that during a procedure one of the containers may empty before the others. This is not a problem if there is just one insulated container.

Detailed Description

In the following description, like elements in the drawings are identified by like numerals, and are differentiated as necessary by appending a letter to the numeral.

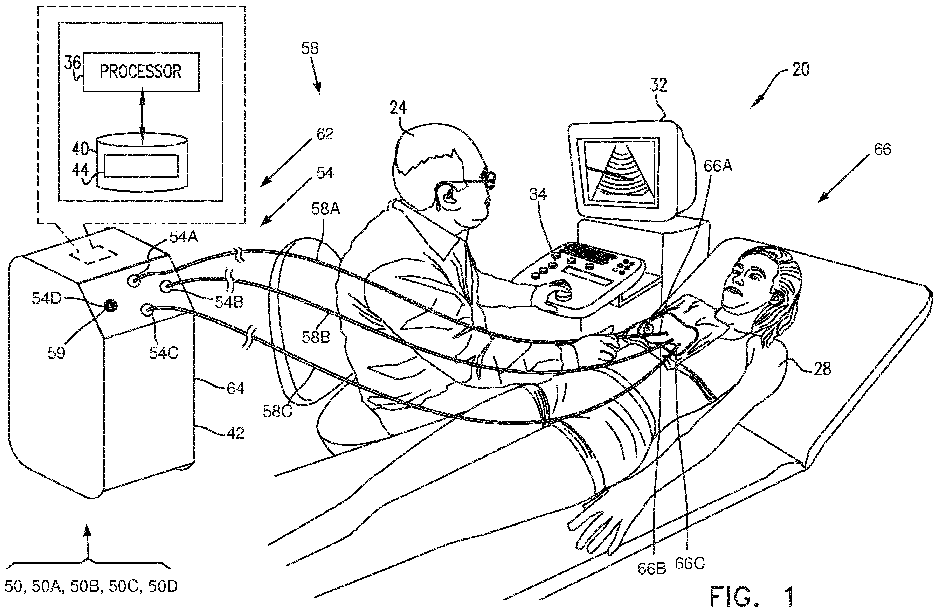

Reference is now made to Fig. 1, which is a schematic illustration of an apparatus 20 being used for a multi-probe cryogenic procedure, according to an embodiment of the present invention. By way of example the procedure assumed in the following description is on a breast tumor, but it will be understood that apparatus 20 may be used for other procedures, such as on a prostate or kidney tumor, and all such procedures are considered to be comprised within the scope of the present invention.

The procedure is performed by a physician 24 on a patient 28, and the physician is able to observe results of the procedure on a display 32 comprised in apparatus 20. The physician is also able to interact with elements of apparatus 20 via a keypad or pointing device 34. (Typically the procedure on the breast tumor includes performing a scan, such as an ultrasound, CT (computerized tomography), or MRI (magnetic resonance imaging) scan, of the breast of patient 28, and presenting results of the scan on display 32. The scan is normally performed by a professional, other than physician 24. Details of the scan are not relevant to the present disclosure, and for simplicity the professional is not shown in Fig. 1.)

Apparatus 20 comprises a console 42, described in more detail below, and the apparatus is controlled by a processor 36, coupled to a memory 40 wherein is stored software 44 for operation of the apparatus. Processor 36 and memory 40 are typically installed in console 42. Software 44 in memory 40 may be downloaded to the processor in electronic form, over a network, for example. Alternatively or additionally, the software may be provided on non-transitory tangible media, such as optical, magnetic, or electronic storage media.

Console 42 houses, within an external casing 64, a plurality of substantially similar submerged cryogenic pumps 50, each couplable by respective connectors 54 in the console to respective cryogenic probes 58. In the present description console 42 is assumed to house four pumps 50A, 50B, 50C, 50D, respectively attached to connectors 54A, 54B, 54C, 54D. However, it will be appreciated that in other embodiments there may be other numbers of pumps and connectors, so long as there are at least two of each. As illustrated in Fig. 1, physician 24 is assumed to couple three probes 58A, 58B, 58C, generically referred to as probes 58, to connectors 54A, 54B, 54C; no probe is coupled to a fourth connector 54D, so that a plug 59 is inserted into the fourth connector to isolate its connecting pump from the atmosphere.

Each probe 58 has a proximal end 62 and a distal end 66. Physician 24 inserts and manipulates distal ends 66A, 66B, and 66C of their respective probes so that they are positioned correctly, typically using the scan referred to above. The physician is then able, typically using device 34, to activate pumps 50A, 50B, 50C to begin transferring cryogenic fluid to distal ends 66A, 66B, and 66C, so as to chill tissue in proximity to the distal ends. The cryogenic fluid is initially stored within console 42 in the form of a liquid, but during the procedure the fluid may change from a liquid to a liquid/gas mixture, or even to a completely gaseous state. Except where otherwise stated, the cryogenic fluid is herein assumed, by way of example, to comprise liquid or gaseous nitrogen. However, it will be understood that other cryogenic fluids, such as cryogenic argon, may be used in apparatus 20, and all such cryogenic fluids are assumed to be comprised within the scope of the present invention.

Pumps 50 may comprise any kind of submergible cryogenic pump known in the art, typically a reciprocating pump such as a bellows pump, a plunger pump, or a piston pump. In the description hereinbelow pumps 50 in console 42 are assumed, by way of example, to be piston pumps, but the description may be altered, *mutatis mutandis,* to accommodate different pumps.

The cryogenic procedure referred to above is typically performed in two phases: a first phase in which a distal end 66 of a probe 58 is reduced in temperature to an initial temperature, typically between approximately -10°C and approximately -30°C, and a second phase in which the distal end temperature is further reduced to a subsequent temperature lower than the initial temperature. In one embodiment the subsequent temperature is approximately - 160±10°C. As is described further below, each pump 50 may act independently, so that each pump may at any time during a procedure be in one of the two phases.

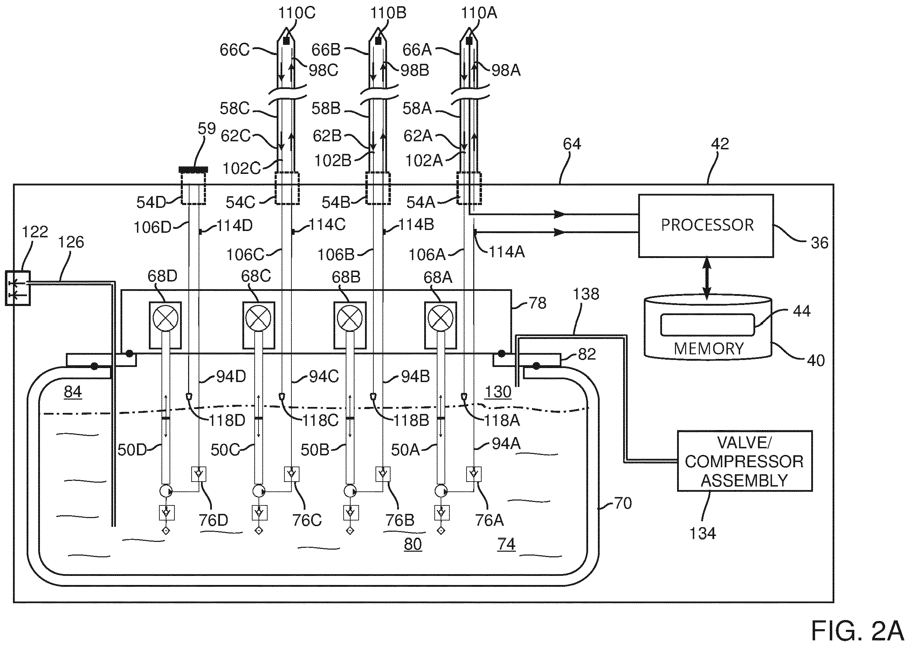

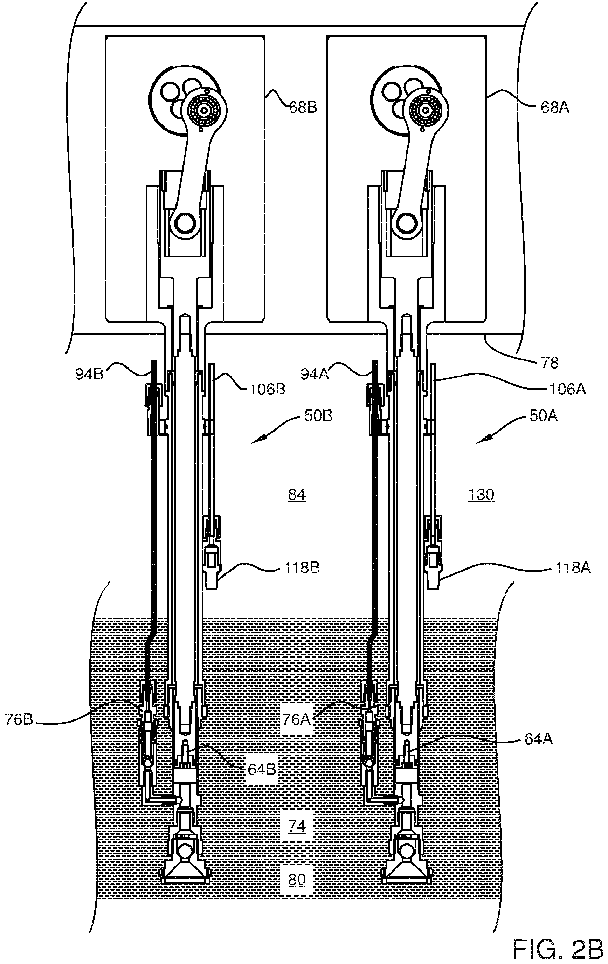

Fig. 2A is a schematic diagram illustrating the structure and operation of console 42, and Fig. 2B is a schematic diagram of two piston pumps operating in the console, according to an embodiment of the present invention. For clarity, in Fig. 2A pumps 50 are shown as rectangles with minimal detail; details of pumps 50 are illustrated in the drawing of pumps 50A, 50B in Fig. 2B.

In Fig. 2A casing 64 of console 42 is shown schematically as a rectangle. As shown, pumps 50A, 50B, 50C, 50D are disposed, i.e., are mounted, within a single insulated container 70, typically a double-walled Dewar having a vacuum between the two walls. Container 70 holds a liquid cryogen 74 within a lower region 80 of the container, and the pumps are mounted so as to be immersed in cryogen. Each of the pumps is driven by a respective motor 68A, 68B, 68C, 68D fixed to a pump-support 78. Pump-support 78 is mounted on a Dewar cover 82, so that, except as described below, container 70 is effectively sealed from the atmosphere. The sealing enables the cryogen within the container to be maintained at a pressure above atmospheric, in one embodiment at approximately 0.5 atmosphere above atmospheric pressure. Maintaining the cryogen above atmospheric pressure prevents liquid cryogen from vaporizing as pumps 50 operate.

Pumps 50 are coupled to respective connectors 54 by outgoing lumens 94, which transfer cryogenic fluid from the pumps to the connectors. The fluid then transfers to corresponding lumens 98 and distal ends 66 in probes 58. Cryogenic fluid returns from distal ends 66 to container 70 via incoming lumens 102 in probes 58, connectors 54, and lumens 106 in console 42. Lumens 106 typically terminate in a liquid/gas separator 118. The lumens are typically formed of concentric cylinders or tubes, but for simplicity in Fig. 2A the lumens are shown as lines. Each distal end 66 has a temperature sensor 110, and the outgoing lumens also have a pressure sensor 114. The respective temperatures measured by sensors 110, and the pressures measured by sensors 114, are provided to processor 36. For clarity, only connections from sensor 110A and 114A to processor 36 are shown in Fig. 2A.

As shown in Fig. 2B for pumps 50A, 50B, each pump 50 comprises a piston 64 which, when activated by motor 68, expels cryogenic fluid from region 80 via a check valve 76 to outgoing lumen 94.

Casing 42 comprises a connector 122, which is coupled to tubing 126, and which, together with the tubing, is used to fill container 70 with cryogen, or to remove cryogen from the container. Liquid cryogen 74 vaporizes to form gaseous cryogen 130 in an upper region 84 of container 70, and the gaseous cryogen can be removed, or held at a regulated pressure, by a valve/compressor assembly 134, which may be controlled by processor 36. Assembly 134 is connected by tubing 138 to gaseous cryogen 130.

Assembly 134 typically comprises a relief valve, which opens to the atmosphere if the pressure of gaseous cryogen 130 exceeds a preset safety limit. The assembly may be used to assist in filling container 70 with cryogen, via connector 122, by removal of gaseous cryogen 130 from the container, so decreasing its pressure. The assembly may also be used to assist in removal of liquid cryogen from container 70, via connector 122, by increasing the pressure of gaseous cryogen 130.

Processor 36 receives the signals from temperature sensors 110 and pressure sensors 114, and in response to these signals the processor generates respective control signals for motors 68, so as to determine the flow rate of cryogen from respective pumps 50. It will be understood that the processor determines the flow rates of each pump independently of the other pumps, i.e., the flow rate for pump 50A is determined by signals from sensor 110A and 114A; the flow rate for pump 50B is determined by signals from sensor 110B and 114B; the flow rate for pump 50C is determined by signals from sensor 110C and 114C; and (when the pump is connected to a probe) the flow rate for pump 50D is determined by signals from sensor 110D and 114D.

It will be appreciated that the pumps in container 70 are physically independent of each other, i.e., the pumps and their associated probes have no common elements.

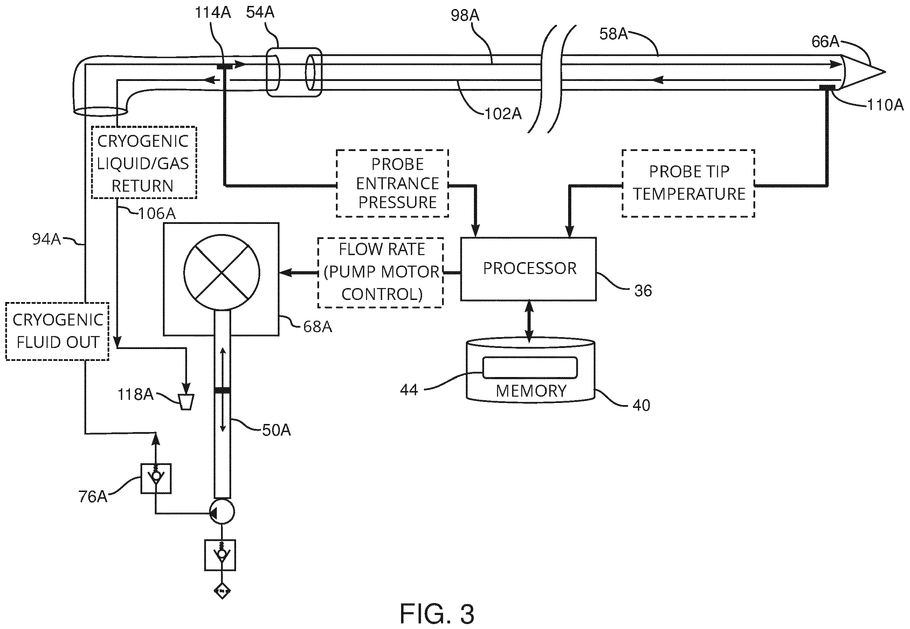

Fig. 3 is a schematic block diagram indicating how processor 36 controls the flow rate of pump 50A, according to an embodiment of the present invention. The diagram illustrates how the elements associated with pump 50A are connected together, and illustrates the flow of cryogenic fluid, and the transfer of signal data, between the elements. While the following description applies to pump 50A, it will be understood that substantially the same description applies, *mutatis mutandis,* to other pumps in container 70.

Processor 36 controls operation of pump 50A by providing pump motor control signals to motor 68A. On activation, the motor operates pump 50A so that cryogenic fluid is expelled, at a flow rate which depends on the revolution rate of the motor, from container 70. Typically, the flow rate of the expelled cryogenic fluid is directly proportional to the motor's revolution rate, so that large revolution rates correspond to large flow rates, and small revolution rates correspond to small flow rates. Flow rate is herein also termed rate of delivery.

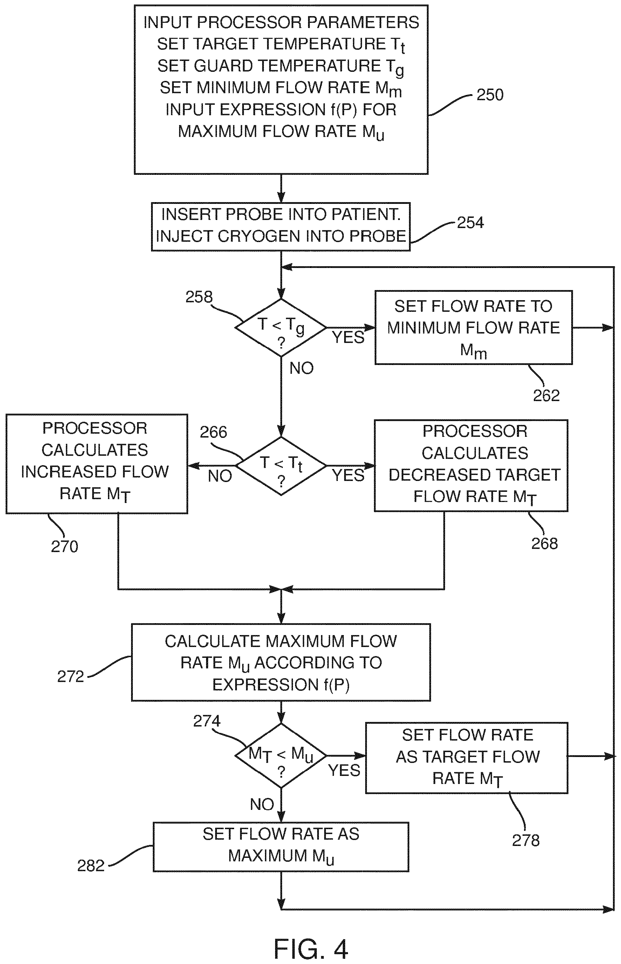

Processor 36 uses the signals from pressure sensor 114A and temperature sensor 110A to operate an algorithm, described below with respect to Fig. 4, which enables the processor to generate an output signal controlling pump motor 68A. The output signal that is transferred to the pump motor controls the revolution rate of the motor and thus the flow rate of the cryogenic fluid expelled from container 70.

The expelled cryogenic fluid flows via check valve 76A, outgoing lumen 94A, lumen 98A of probe 58A, to distal end 66A of the probe. The cryogenic fluid returns from distal end 66A, typically as a liquid/gas mixture, via lumens 102A, 106A, and separator 118A to container 70.

Fig. 4 is a flowchart of operations taken by processor 36 in executing an algorithm to operate pump 50A during the first phase of the procedure of Fig. 1, according to an embodiment of the present invention. As stated above, processor 36 operates any given pump 50 independently of, and simultaneously with, other pumps 50. Thus, processor 36 may operate any other pumps 50 according to the flowchart, *mutatis mutandis,* of Fig. 4. Alternatively or additionally, processor 36 may operate any of pumps 50 to provide cryogen to respective pump distal ends, by any method known in the art for cryogenic cooling of a distal end. Further alternatively or additionally, some of the pumps may not be activated, i.e., there is no flow from these non-activated pumps. Such non-activated pumps may be present when the respective probes of the pumps are inserted or not inserted into a living subject.

In the description of the flowchart, processor 36 is assumed to comprise a PID (proportional-integral-derivative) controller, having an output given by equation (1) :

$$u(t) = K_c \left[ e(t) + \frac{1}{t_i} \int_0^t e(t)dt + t_d \frac{de(t)}{dt} \right] \qquad (1)$$

where u(t) is the control signal, output by the processor at time t, and

$K_c$, $t_i$, $t_d$ are respective coefficients of the proportional, integral, and differential terms.

A person having ordinary skill in the art will be able to adapt the flowchart description without undue experimentation, *mutatis mutandis,* for processors other than PID controllers.

In a first step 250, parameters for processor 36, herein, as stated above, assumed to comprise a PID controller, are input to the processor. In the following description the PID controller is assumed to have coefficient values of $K_c$ = 0.1 and $t_i$=0.05, and $t_d$=0, i.e., processor 36 operates as a PI controller, and those having ordinary skill in the art will be able to adapt the description, without undue experimentation, for other values of $K_c$, $t_i$, and $t_d$.

In step 250 processor 36 is provided with a target temperature $T_t$, which is a nominal temperature to which distal end 66A is cooled. Temperature $T_t$ is typically in a range from -10°C to -50°C, although in some embodiments $T_t$ is outside this range. Processor 36 is also provided with a guard temperature $T_g$, which is a temperature less than $T_t$, that acts as a nominal temperature limit for the distal send. In one embodiment when $T_t$ = -20°C, $T_g$ = -23°C.

The processor also sets a minimum flow rate $M_m$, corresponding to a minimum speed that the processor applies to pump motor 68A. In one embodiment the minimum motor speed is set at 224 rpm.

In initial step 250 the processor is also provided with a function f(P) that the processor uses to calculate a maximum flow rate $M_u$, corresponding to a maximum speed to be applied to the pump motor, i.e.,

$$M_u = f(P) \qquad (2)$$

where f is a function of pressure P measured by pressure sensor 114A.

In an embodiment of the invention f(P) is configured so that if P increases the maximum flow rate $M_u$ falls, and if P decreases $M_u$ increases. In the embodiment both derivatives $\frac{dM_u}{dP}$, and $\frac{dP}{dt}$ are negative.

In a disclosed embodiment equation (2) is set as equation (3):

$$M_u = K_1 \cdot \left( 1 - \frac{P}{P_{arb}} \right) \qquad (3)$$

where $P_{arb}$ is a parametric pressure having a fixed value that is set to be greater than a critical pressure of the cryogenic fluid being used by apparatus 20; and

$K_1$ is a proportionality constant used to convert the expression on the right side of equation (3) to the units of $M_u$.

From equation (3) the gradient $\dfrac{dM_u}{dP}$, corresponding to the first derivative of the equation, is given by equation (3a) :

$$\frac{dM_u}{dP} = -\frac{K_1}{P_{arb}} \qquad (3a)$$

In a procedure initialization step 254, physician 24 inserts probe 58A into patient 28, and activates pump 50A to inject cryogenic fluid into the probe.

In a first decision step 258, the processor compares the temperature T measured by sensor 110A with guard temperature $T_g$, by evaluating the expression

$$T < T_g \qquad (4)$$

If expression (4) returns positive, i.e., temperature T is less than guard temperature $T_g$, then the processor proceeds to a first set flow rate step 262, wherein the processor sets the flow rate to the minimum flow rate $M_m$. Control then returns to decision step 258.

If expression (4) returns negative, i.e., temperature T is greater than or equal to the guard temperature, then control continues to a second decision step 266. In step 266 the processor compares temperature T with the target temperature $T_t$ by evaluating the expression

$$T < T_t \qquad (5)$$

If step 266 returns positive, i.e., temperature T is less than target temperature $T_t$, then in a decrease rate step 268, the processor calculates a decreased target flow rate $M_T$.

If step 266 returns negative, i.e., temperature T is greater than or equal to target temperature $T_t$, then in an increase rate step 270, the processor calculates an increased target flow rate $M_T$.

In steps 268 and 270, the processor evaluates u(t) according to equation (1), and determines the target flow rate $M_T$ according to equation (6):

$$M_T = K_2 \cdot u(t) \qquad (6)$$

where $K_2$ is a proportionality constant used to convert u(t) to the units of flow rate.

Control from steps 268 and 270 continues at a maximum flow rate step 272.

In step 272 the processor accesses the value of pressure P provided by pressure sensor 114A, and uses the value to calculate the maximum flow rate $M_u$ according to equation (2).

In a third decision step 274, the processor compares the target flow rate and the maximum flow rate, by evaluating the expression

$$M_T < M_u \qquad (7)$$

If expression (7) returns positive, i.e., the target flow rate $M_T$ is less than the maximum flow rate $M_u$, then the processor proceeds to a second set flow rate step 278, wherein the processor sets the flow rate to the target flow rate.

If expression (7) returns negative, i.e., flow rate $M_T$ is equal to or greater than maximum flow rate $M_u$, then the processor proceeds to a third set flow rate step 282, wherein the processor sets the flow rate to the maximum flow rate.

The flowchart of Fig. 4 reiterates by control from the flow rate steps 262, 278, and 282 returning to decision step 258.

The description of the flowchart of Fig. 4 is for pump 50A, so that the parameters defined in step 250, as well as the temperatures, flow rates, and function f(P) defined in the step apply for operating this pump. As stated above, the description may be applied, *mutatis mutandis,* for operating other pumps in container 70; since the pumps are operated independently of each other, one or more of the parameters, temperatures, flow rates, and function f(P) defined in step 250 may be different for each pump, or they may be the same.

For example, $T_t$ for pump 50A may be set at -20°C, for pump 50B $T_t$ may also be set at -20°C, and for pump 50C $T_t$ may be set at -25°C.

As described above processor 36 is configured to operate all pumps in container 70, and in the case when the processor is a PID processor, the values of $K_c$, $t_i$, $t_d$ are typically the same. However, this is not a necessity, and there may be cases when at least one of the values $K_c$, $t_i$, $t_d$ is different from that for other pumps.

As stated above, the flowchart of Fig. 4 is for the first phase of the cryogenic procedure, and this applies for any given pump 50. Once the first phase has been completed, the second phase of the procedure may be initiated. Since pumps 50 are operated independently, it will be understood that at any given time during a procedure any given pump 50 may be in the first phase of the procedure or in the second phase.

While the description above assumes that processor 36 is typically a single processor, it will be understood that each pump may have its own processor, and those having ordinary skill in the art will be able to adapt the description, *mutatis mutandis,* for multiple processors.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

**Claims**

1. Apparatus, comprising:

   a single insulated container, configured to contain a cryogen;
   a first pump and a second pump, both disposed in the single insulated container and configured to pump the cryogen from the single insulated container while immersed in the cryogen; and
   a first probe and a second probe, having respective first and second proximal ends coupled to receive concurrently the cryogen pumped by the first pump and the second pump, respectively, and having respective first and second distal ends, which are chilled by the cryogen received through the first and second proximal ends and are configured to be inserted into a body of a living subject.

2. The apparatus according to claim 1, wherein the first pump and the second pump are respectively selected from a group comprising a bellows pump, a plunger pump, and a piston pump.

3. The apparatus according to claim 1, wherein the first pump and the second pump are not connected.

4. The apparatus according to claim 1, wherein the cryogen pumped by the first pump is at a first rate of flow, and wherein the cryogen pumped by the second pump is at a second rate of flow, and wherein the first and second rates of flow are independent of each other.

5. The apparatus according to claim 4, wherein the first distal end comprises a first temperature sensor and the second distal end comprises a second temperature sensor, and wherein the first rate of flow is in response to a first temperature measured by the first temperature sensor and the second rate of flow is in response to a second temperature measured by the second temperature sensor.

6. The apparatus according to any of claims 1-4, and comprising a processor configured to operate the first pump and the second pump.

7. A method, comprising:

   providing a single insulated container configured to contain a cryogen;
   disposing a first pump and a second pump in the single insulated container, and configuring the pumps to pump the cryogen from the single insulated container while immersed in the cryogen; and
   coupling a first probe and a second probe respectively to the first pump and the second pump, wherein the first probe and the second probe have respective first and second proximal ends coupled to receive concurrently the cryogen pumped by the first pump and the second pump, respectively, and have respective first and second distal ends, which are chilled by the cryogen received through the first and second proximal ends and are configured to be inserted into a body of a living subject

8. The method according to claim 7, wherein the first pump and the second pump are respectively selected from a group comprising a bellows pump, a plunger pump, and a piston pump.

9. The method according to claim 7, wherein the first pump and the second pump are not connected.

10. The method according to claim 7, wherein the cryogen pumped by the first pump is at a first rate of flow, and wherein the cryogen pumped by the second pump is at a second rate of flow, and wherein the first and second rates of flow are independent of each other.

11. The method according to claim 10, wherein the first distal end comprises a first temperature sensor and the second distal end comprises a second temperature sensor, and wherein the first rate of flow is in response to a first temperature measured by the first temperature sensor and the second rate of flow is in response to a second temperature measured by the second temperature sensor.

12. The method according to any of claims 7-10, and comprising operating the first pump and the second pump by a single processor.

FIG. 1

EP 4 285 849 A1

FIG. 2A

FIG. 2B

FIG. 3

FIG. 4

| | Europäisches Patentamt European Patent Office Office européen des brevets | EUROPEAN SEARCH REPORT | Application Number EP 23 17 2909 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 550 666 B1 (CRYOMEDICAL SCIENCES INC [US]) 13 January 1999 (1999-01-13) * paragraphs [0033] – [0047], [0056], [0068], [0081] – [0083], [0097], [0107], [0110]; figures 6, 7B, 12 * | 1-12 | INV. A61B18/02 ADD. A61B18/00 |
| X | US 2010/256622 A1 (BAUST JOHN M [US] ET AL) 7 October 2010 (2010-10-07) * paragraphs [0019], [0024], [0035] – [0039]; figure 4 * | 1,2,7,8 | |
| X | US 2020/297403 A1 (KOCHAVI EYAL [IL]) 24 September 2020 (2020-09-24) * paragraphs [0277], [0319] – [0322]; figure 11 * | 1,7 | |
| A | US 2021/244457 A1 (HILLELI RON [IL] ET AL) 12 August 2021 (2021-08-12) * paragraph [0051]; figure 2 * | 2,8 | |
| A | US 2006/100495 A1 (SANTOIANNI DOMENIC [CA] ET AL) 11 May 2006 (2006-05-11) * paragraphs [0053], [0059], [0083], [0084], [0102], [0116], [0117]; figures 1, 25 * | 1-12 | TECHNICAL FIELDS SEARCHED (IPC) A61B |
| A | US 5 860 971 A (CLARKE BRIAN KEVIN RODERICK [GB]) 19 January 1999 (1999-01-19) * figure 5 * | 1-12 | |
| A | WO 2019/077508 A1 (GALIL MEDICAL INC [US]) 25 April 2019 (2019-04-25) * the whole document * | 1-12 | |
| A | US 6 039 730 A (RABIN YOED [US] ET AL) 21 March 2000 (2000-03-21) * figure 4 * | 1-12 | |

-/--

The present search report has been drawn up for all claims

| Place of search | Date of completion of the search | Examiner |
|---|---|---|
| The Hague | 26 September 2023 | Bois, Nadège |

EPO FORM 1503 03.82 (P04C01)

page 1 of 2

**EUROPEAN SEARCH REPORT**

Europäisches Patentamt
European Patent Office
Office européen des brevets

Application Number

EP 23 17 2909

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 6 190 378 B1 (JARVINEN PHILIP O [US]) 20 February 2001 (2001-02-20) * figures 1, 2 * ----- | 1-12 | |

TECHNICAL FIELDS SEARCHED (IPC)

The present search report has been drawn up for all claims

| Place of search | Date of completion of the search | Examiner |
|---|---|---|
| The Hague | 26 September 2023 | Bois, Nadège |

EPO FORM 1503 03.82 (P04C01)

## ANNEX TO THE EUROPEAN SEARCH REPORT
## ON EUROPEAN PATENT APPLICATION NO.

EP 23 17 2909

This annex lists the patent family members relating to the patent documents cited in the above-mentioned European search report.
The members are as contained in the European Patent Office EDP file on
The European Patent Office is in no way liable for these particulars which are merely given for the purpose of information.

26-09-2023

| Patent document cited in search report | | | Publication date | Patent family member(s) | | | Publication date |
|---|---|---|---|---|---|---|---|
| EP 0550666 | B1 | | 13-01-1999 | AT | E175561 | T1 | 15-01-1999 |
| | | | | AU | 649741 | B2 | 02-06-1994 |
| | | | | CA | 2091893 | A1 | 27-03-1992 |
| | | | | EP | 0550666 | A1 | 14-07-1993 |
| | | | | IL | 99479 | A | 10-06-1997 |
| | | | | JP | H06500940 | A | 27-01-1994 |
| | | | | NZ | 239884 | A | 27-09-1994 |
| | | | | TW | 235911 | B | 11-12-1994 |
| | | | | US | 5334181 | A | 02-08-1994 |
| | | | | US | 5674218 | A | 07-10-1997 |
| | | | | WO | 9204872 | A1 | 02-04-1992 |
| | | | | ZA | 917281 | B | 26-08-1992 |
| US 2010256622 | A1 | | 07-10-2010 | US | 2010057067 | A1 | 04-03-2010 |
| | | | | US | 2010256622 | A1 | 07-10-2010 |
| | | | | US | 2016338754 | A1 | 24-11-2016 |
| | | | | US | 2020138500 | A1 | 07-05-2020 |
| US 2020297403 | A1 | | 24-09-2020 | CN | 110461260 | A | 15-11-2019 |
| | | | | CN | 115444540 | A | 09-12-2022 |
| | | | | EP | 3576656 | A1 | 11-12-2019 |
| | | | | EP | 4218637 | A1 | 02-08-2023 |
| | | | | ES | 2936210 | T3 | 15-03-2023 |
| | | | | JP | 7177072 | B2 | 22-11-2022 |
| | | | | JP | 2020518305 | A | 25-06-2020 |
| | | | | JP | 2023011924 | A | 24-01-2023 |
| | | | | US | 2020297403 | A1 | 24-09-2020 |
| | | | | WO | 2018142411 | A1 | 09-08-2018 |
| US 2021244457 | A1 | | 12-08-2021 | CN | 113243983 | A | 13-08-2021 |
| | | | | EP | 3868320 | A1 | 25-08-2021 |
| | | | | ES | 2919851 | T3 | 28-07-2022 |
| | | | | JP | 7219498 | B2 | 08-02-2023 |
| | | | | JP | 2021126513 | A | 02-09-2021 |
| | | | | US | 2021244457 | A1 | 12-08-2021 |
| | | | | US | 2023210575 | A1 | 06-07-2023 |
| US 2006100495 | A1 | | 11-05-2006 | CA | 2598919 | A1 | 05-10-2006 |
| | | | | EP | 1865868 | A2 | 19-12-2007 |
| | | | | US | 2006100495 | A1 | 11-05-2006 |
| | | | | WO | 2006104837 | A1 | 05-10-2006 |
| US 5860971 | A | | 19-01-1999 | NONE | | | |
| WO 2019077508 | A1 | | 25-04-2019 | AU | 2018352315 | A1 | 23-04-2020 |
| | | | | CN | 111565659 | A | 21-08-2020 |

For more details about this annex : see Official Journal of the European Patent Office, No. 12/82

page 1 of 2

**EP 4 285 849 A1**

## ANNEX TO THE EUROPEAN SEARCH REPORT
## ON EUROPEAN PATENT APPLICATION NO.

EP 23 17 2909

This annex lists the patent family members relating to the patent documents cited in  the above-mentioned European search report.
The members are as contained in the European Patent Office EDP file on
The European Patent Office is in no way liable for these particulars which are merely given for the purpose of information.

26-09-2023

| Patent document cited in search report | | Publication date | Patent family member(s) | | Publication date |
|---|---|---|---|---|---|
| | | | EP | 3697330 A1 | 26-08-2020 |
| | | | JP | 7122376 B2 | 19-08-2022 |
| | | | JP | 2021500127 A | 07-01-2021 |
| | | | TW | 201916863 A | 01-05-2019 |
| | | | WO | 2019077508 A1 | 25-04-2019 |
| US 6039730 | A | 21-03-2000 | AU | 3571197 A | 14-01-1998 |
| | | | CA | 2258619 A1 | 31-12-1997 |
| | | | EP | 0917447 A1 | 26-05-1999 |
| | | | JP | 2000513963 A | 24-10-2000 |
| | | | US | 6039730 A | 21-03-2000 |
| | | | US | 6786902 B1 | 07-09-2004 |
| | | | WO | 9749344 A1 | 31-12-1997 |
| US 6190378 | B1 | 20-02-2001 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7083612 B, Littrup
- US 7192426 B, Baust
- US 7422583 B
- US 20040210212 A, Maurice
- US 8784409 B, Robilotto
- US 9316215 B, Mackey
- US 11026737 B, Baust
- US 11060778 B, Jankowsky

- US 11266458 B, Perron
- US 20070244474 A, DeLonzor
- US 20080125764 A, Vancelette
- US 20150126987 A, Semenov
- US 20160249970 A, Yu
- US 20200121498 A, Baust
- US 20210177482 A, Tegg
- US 20210244457 A, Hilleli